# EUROPEAN PATENT APPLICATION

(11) **EP 4 801 240 A1**
(43) Date of publication of application: **02.09.2026**
(21) Application number: 24882513.5
(22) Date of filing: 25.10.2024
(51) Int. Cl.: H10K 30/50, C07D 487/22, H10K 30/40, H10K 30/86, H10K 85/10, H10K 85/30, H10K 85/50

(54) **PHOTOELECTRIC CONVERSION ELEMENT, PHOTOELECTRIC CONVERSION DEVICE, AND METHOD FOR MANUFACTURING PHOTOELECTRIC CONVERSION ELEMENT**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086000
(71) Applicant: Canon Kabushiki Kaisha, Tokyo 146-8501 (JP)
(72) Inventor: MURANAKA Norifumi, Tokyo 146-8501 (JP); OHSAWA Tatsuya, Tokyo 146-8501 (JP); NISHIDA Tsutomu, Tokyo 146-8501 (JP); WATARIGUCHI Kaname, Tokyo 146-8501 (JP); KITAHARA Michitaka, Tokyo 146-8501 (JP)
(74) Representative: TBK
(86) International application number: PCT/JP2024/038173
(87) International publication number: WO 2025/089405

(57) **Abstract**

To provide a photoelectric conversion element having improved photoelectric conversion efficiency, the present invention provides a photoelectric conversion element comprising: a first electrode; a second electrode; and a photoelectric conversion layer arranged between the first electrode and the second electrode, the photoelectric conversion layer containing a crystal having a perovskite structure, wherein the photoelectric conversion element includes a charge-transporting layer arranged between the photoelectric conversion layer and the first electrode, and wherein the charge-transporting layer is formed of a charge-transporting particle and an insulating resin on a surface of the photoelectric conversion layer.

## Description

### [Technical Field]

The present invention relates to a photoelectric conversion element, a photoelectric conversion apparatus, and a method of producing a photoelectric conversion element.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate. Accordingly, cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic.

For example, in Patent Literature 1, there is a description of a technology including incorporating an organic semiconductor and a polymer compound having a glass transition temperature of 100°C or more into a hole-transporting layer to improve its peeling from an electrode. In Non Patent Literature 1, there is a description that conversion efficiency is improved by mixing copper phthalocyanine and a conductive polymer into a hole-transporting layer.

### [Citation List]

### [Patent Literature]

PTL 1: Japanese Patent Laid-Open No. 2018-170382

### [Non Patent Literature]

NPL 1: Q. Hu, et al, Sol. RRL, 2019, 3, 1800264

### [Summary of Invention]

### [Technical Problem]

According to investigations made by the inventors of the present invention, it has been found that there is room for improvement in photoelectric conversion efficiency in each of the photoelectric conversion elements described in Patent Literature 1 and Non Patent Literature 1.

Accordingly, the present invention is directed to providing a photoelectric conversion element having improved photoelectric conversion efficiency. The present invention is also directed to providing a photoelectric conversion apparatus having improved photoelectric conversion efficiency. The present invention is also directed to providing a method of producing a photoelectric conversion element having improved photoelectric conversion efficiency.

### [Solution to Problem]

The above-mentioned provision is achieved by the present invention described below. That is, the present invention is directed to a photoelectric conversion element comprising:
a first electrode;
a second electrode; and a photoelectric conversion layer arranged between the first electrode and the second electrode, the photoelectric conversion layer containing a crystal having a perovskite structure,
wherein the photoelectric conversion element comprises a charge-transporting layer arranged between the photoelectric conversion layer and the first electrode, and
wherein the charge-transporting layer is formed of a charge-transporting particle and an insulating resin on a surface of the photoelectric conversion layer.

The present invention is also directed to a photoelectric conversion apparatus comprising the photoelectric conversion element.

The present invention is also directed to a method of producing a photoelectric conversion element, the method comprising the steps of:
forming a first electrode;
forming a second electrode;
forming a photoelectric conversion layer containing a crystal having a perovskite structure between the first electrode and the second electrode; and
forming a charge-transporting layer between the photoelectric conversion layer and the first electrode,
wherein the charge-transporting layer contains a charge-transporting particle and an insulating resin, and is formed on a surface of the photoelectric conversion layer.

### [Advantageous Effects of Invention]

According to the present invention, the photoelectric conversion element having improved photoelectric conversion efficiency can be provided.

### [Brief Description of Drawings]

[Fig. 1]

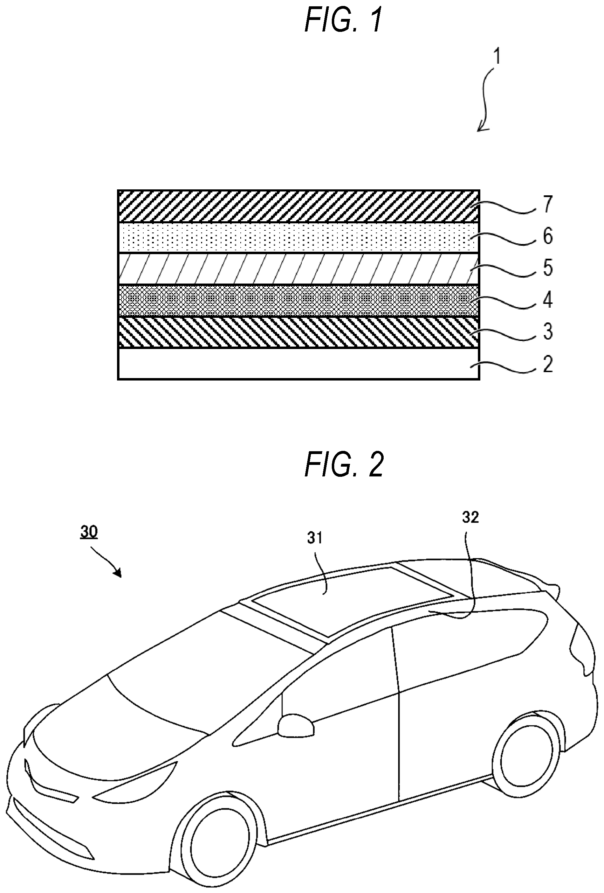

   Fig. 1 is a schematic diagram of the layer configuration in a thickness direction of a photoelectric conversion element according to a first embodiment of the present invention.
[Fig. 2]
   Fig. 2 is a perspective view for schematically illustrating a moving body including the photoelectric conversion element according to one embodiment of the present invention.
[Fig. 3]

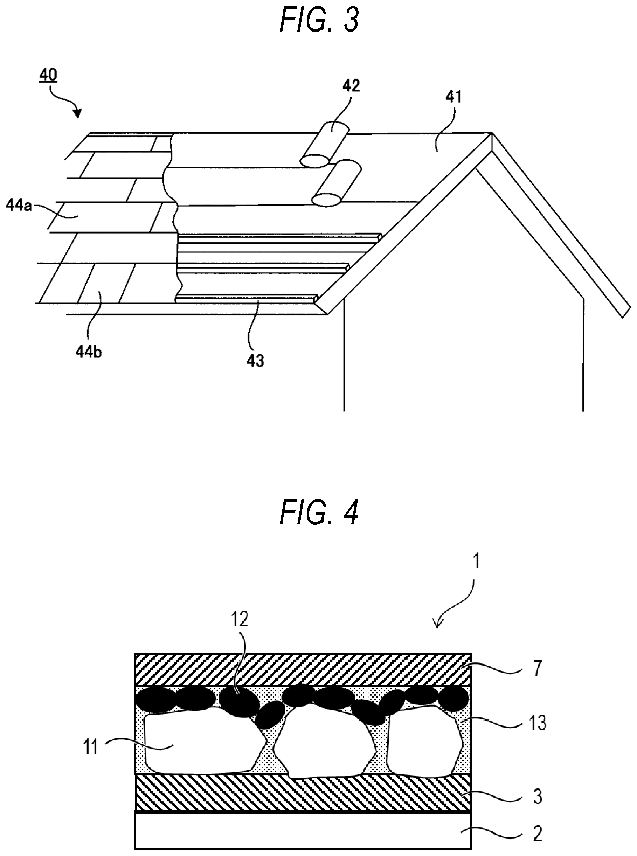

   Fig. 3 is a perspective view for schematically illustrating a building material including the photoelectric conversion element according to one embodiment of the present invention.
[Fig. 4]
   Fig. 4 is a schematic sectional view of the photoelectric conversion element according to one embodiment of the present invention.

### [Description of Embodiments]

### <First Embodiment>

A first embodiment is directed to a photoelectric conversion element. The photoelectric conversion element of the present invention comprises:
a first electrode;
a second electrode; and a photoelectric conversion layer arranged between the first electrode and the second electrode, the photoelectric conversion layer containing a crystal having a perovskite structure,
wherein the photoelectric conversion element comprises a charge-transporting layer arranged between the photoelectric conversion layer and the first electrode, and
wherein the charge-transporting layer is formed of a charge-transporting particle and an insulating resin on a surface of the photoelectric conversion layer.

As a result of investigations, the inventors of the present invention have found that a photoelectric conversion element having excellent conversion efficiency is obtained by incorporating the above-mentioned charge-transporting layer. The reason therefor is conceived to be as described below.

The photoelectric conversion layer containing a crystal having a perovskite structure has unevenness on a surface of the crystal. In addition, a gap may be present between crystal grains. When oxygen or the like is present in the unevenness or the gap, deterioration tends to proceed, and hence the unevenness or the gap is preferably filled with a charge-transporting layer or the like. However, when a low-resistance charge-transporting layer is formed on the surface of the photoelectric conversion layer containing the crystal having a perovskite structure, surfaces up to the side surfaces of the unevenness and the gap are covered with a low-resistance material with respect to the flow of charge. As a result, a state in which an electron and a hole easily recombine is established, and the state may lead to a reduction in photoelectric conversion efficiency. In addition, when a high-resistance charge-transporting layer is formed on the surface of the photoelectric conversion layer containing the crystal having a perovskite structure, the exchange of charge is hindered, and hence the conversion efficiency of the photoelectric conversion element may reduce.

Meanwhile, when a charge-transporting layer is formed of a charge-transporting particle and an insulating resin on the crystal having a perovskite structure, the charge-transporting particle and the insulating resin are present independently. As a result, the insulating resin preferentially penetrates into the side surfaces of the unevenness and gap of the crystal having a perovskite structure, and hence the recombination of an electron and a hole occurring there is suppressed.

At the same time, it is conceived that, when the charge-transporting particle is present on the perovskite crystal, charge transportation efficiency from the perovskite crystal to the first electrode is maintained without the insulating resin becoming an obstacle to charge transportation.

### <Charge-transporting Particle and Insulating Resin>

In the photoelectric conversion element of the present invention, the charge-transporting layer is formed of the charge-transporting particle and the insulating resin on the surface of the photoelectric conversion layer. In the present invention, the average particle diameter of the charge-transporting particle in the charge-transporting layer is preferably 1.0×10¹ to 3.0×10² nm. When the average particle diameter falls within the above-mentioned range, the penetration of the charge-transporting particle into a gap between perovskite crystal grains can be suppressed and the uniformity of a film is maintained, and hence the loss of charge transportability can be suppressed. The particle diameter of the charge-transporting particle in the charge-transporting layer may be determined as a volume-average particle diameter from a particle size distribution determined by an image imaging method using a scanning electron microscope (SEM).

In addition, specific material examples of the charge-transporting particle include a phthalocyanine pigment, an azo pigment, a lake pigment, a quinacridone pigment, a dioxazine pigment, a perylene pigment, and an isoindolinone pigment.

In the present invention, the charge-transporting particle is preferably a particle containing a cyclic conjugated compound in which a plurality of pyrrole rings are bonded by conjugated bonds. When the cyclic conjugated compound in which the plurality of pyrrole rings are bonded by covalent bonds is formed into a charge-transporting particle, high charge transportability is exhibited.

In the present invention, the charge-transporting particle is preferably a particle containing a phthalocyanine compound, more preferably a particle having a structure represented by the following formula (Pc-2). The charge-transporting particle can more efficiently transport charge generated in the photoelectric conversion layer.

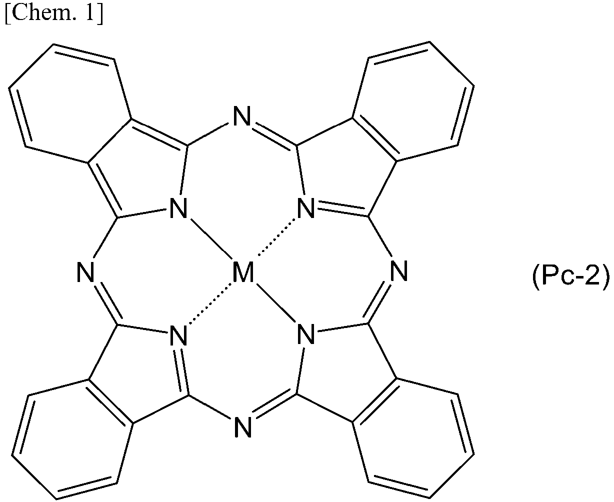

M in the formula (Pc-2) represents H₂, a metal atom having a ligand, or a metal atom free of a ligand.

The structure of a chemical substance such as the charge-transporting particle of the present invention may be identified by a nuclear magnetic resonance (NMR) method.

In particular, when M in the formula (Pc-2) represents H₂, the formula (Pc-2) is represented by the following formula (Pc-1).

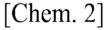

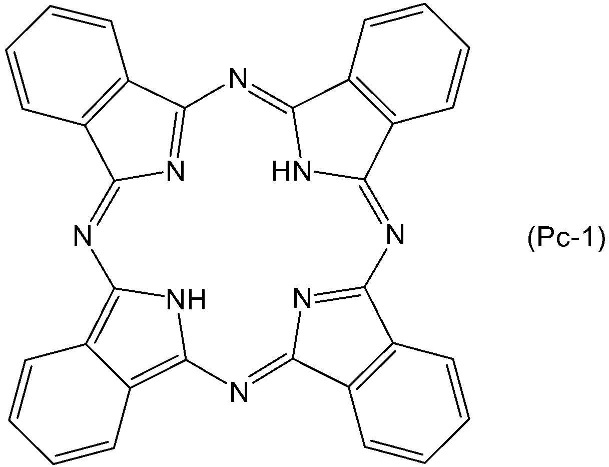

Specific examples of the insulating resin include a polyacetal resin, an acrylic resin, a polyarylate resin, a polycarbonate resin, a polyvinyl acetate resin, a polyester resin, a polyamide resin, a polyurethane resin, and a polystyrene resin.

In the present invention, the glass transition temperature of the insulating resin is preferably 95°C or less. When the glass transition temperature falls within this range, the insulating resin is easily brought into close contact with the charge-transporting particle, and hence a more effective charge distribution can be formed. The glass transition temperature may be determined with a differential scanning calorimeter (DSC).

In the present invention, the insulating resin is preferably a polyvinyl acetal resin or a polyvinyl butyral resin. When the insulating resin is one of these insulating resins, the insulating resin is easily brought into close contact with the charge-transporting particle, and hence a more effective charge distribution can be formed.

In the present invention, it is preferred that the charge-transporting layer contain an aromatic ring compound having a hydroxy group, the aromatic ring compound being different from the charge-transporting particle (pigment) and the insulating resin. When the charge-transporting layer contains the aromatic ring compound having a hydroxy group, the charge-transporting particle and the insulating resin are easily brought into contact with each other, and hence a more effective charge distribution can be formed.

In the present invention, the photoelectric conversion element may comprise a second charge-transporting layer between the first electrode and the charge-transporting layer. When the photoelectric conversion element comprises the second charge-transporting layer, the transfer of carriers to an electrode may be facilitated.

The effects of the present invention can be achieved when the respective configurations synergistically exhibit effects on each other as in the mechanism as described above.

The present invention is described in detail below by way of illustrative embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element. The analysis of each layer may be performed by peeling and removing a layer from a completed photoelectric conversion element to expose the layer to be analyzed. In the present invention, for the quantification of a volume ratio, the area ratio of an exposed surface or a cross section is used as the volume ratio of the layer.

Fig. 1 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes (the second electrode 3 and the first electrode 7), and may not be formed in some cases. A form in which the plurality of electron-transporting layers 4 and photoelectric conversion layers 5 are laminated may be adopted. Such form may also be referred to as "tandem structure." The respective members are described below. In addition, the photoelectric conversion element may be produced in the order of the first electrode 7, the charge-transporting layer 6, the photoelectric conversion layer 5, the electron-transporting layer 4, and the second electrode 3 on the substrate 2.

### [Photoelectric Conversion Element]

The photoelectric conversion element of the present invention is characterized by comprising: the first electrode; the second electrode; the photoelectric conversion layer arranged between the first electrode and the second electrode, the photoelectric conversion layer containing the crystal having a perovskite structure; and the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may comprise the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

The photoelectric conversion element of the present invention comprises the first electrode and the second electrode. A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers.

Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion element of the present invention comprises the photoelectric conversion layer arranged between the first electrode and the second electrode, the photoelectric conversion layer containing a crystal having a perovskite structure. The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚNₘHₙ ("p", "m", and "n" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are layered as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4]. "n" represented in each of the following general formulae represents a positive integer.

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule, which may have a substitute, or of a metal. Specifically, ethylammonium, propylammonium, n-butylammonium, n-hexylammonium, n-octylammonium, 1,6-hexanediammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I_{16,} (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

In addition, the photoelectric conversion layer according to the present invention may contain a material except the crystal having an organic-inorganic perovskite structure to the extent that the photoelectric conversion efficiency and the charge transportability are not impaired.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

In the present invention, the surface roughness Ra of the crystal having a perovskite structure of the photoelectric conversion layer is preferably 10 to 200 nm. When the surface roughness Ra is 10 nm or more, the recombination-suppressing effect of the insulating resin is more easily exhibited. In addition, when the surface roughness Ra is larger than 200 nm, the charge-transporting particle tends to penetrate to the second electrode side of the photoelectric conversion layer, and the recombination-suppressing effect may reduce.

### [Charge-transporting Layer]

In the photoelectric conversion element of the present invention, the charge-transporting layer is arranged between the photoelectric conversion layer and the first electrode, and the charge-transporting layer is formed of a charge-transporting particle and an insulating resin on the surface of the photoelectric conversion layer. In the photoelectric conversion element of the present invention, the insulating resin is preferably arranged between the crystals each having a perovskite structure of the photoelectric conversion layer. Each item such as the charge-transporting particle or the insulating resin is as described above.

In the present invention, the charge-transporting layer contains the charge-transporting particle, which is a P-type semiconductor, and the insulating resin, and the volume of the charge-transporting particle in the charge-transporting layer is preferably 5 to 30 times with respect to the volume of the insulating resin in the charge-transporting layer. The insulating resin has a volume resistivity of 10⁸ Ω·cm or more.

The layer thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the liquid on the photoelectric conversion layer, and drying the coating film. Examples of the solvent to be used for the coating liquid include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

In the present invention, the photoelectric conversion element may further comprise the second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound preferably has an aromatic ring from the viewpoint of the compatibility of a film interface, and Spiro-OMeTAD, PTAA, or a phthalocyanine compound is particularly preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. In particular, tin oxide may be obtained through the reaction of tin(II) chloride, tin(IV) chloride, tin(II) chloride dihydrate, or tin(IV) chloride pentahydrate.

A preferred lower limit of the thickness of the electron-transporting layer 4 is 1 nm, and a preferred upper limit thereof is 2,000 nm. When the thickness of the electron-transporting layer 4 is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### [Control of Particle Diameter of Charge-transporting Particle]

The particle diameter of the charge-transporting particle may be changed by dispersing the coating liquid for a charge-transporting layer with a paint shaker, and the particle diameter may be reduced by increasing a dispersion time. In addition, the particle diameter may be reduced by further subjecting the coating liquid for a charge-transporting layer to a centrifuge.

### <Application Examples>

Application examples of the present invention are directed to a photoelectric conversion apparatus, a moving body, and a building material.

### [Photoelectric Conversion Apparatus]

A photoelectric conversion apparatus of the present invention comprises the above-mentioned photoelectric conversion element. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage.

In addition, the photoelectric conversion apparatus comprises the photoelectric conversion element of the present invention and an inverter. The inverter may be a converter for converting a DC voltage to an AC voltage. The photoelectric conversion apparatus may comprise an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

A moving body of the present invention includes the above-mentioned photoelectric conversion element. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the above-mentioned photoelectric conversion element. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a higher thermal conductivity than the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

In addition to the application examples described above, the following application examples are given: portable devices, such as a calculator, a sensor, and a small solar panel; wearable devices, such as a glasses-type terminal, a watch-type terminal, and a portable medical device; sheet structures supported by a plurality of frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### <Second Embodiment>

A second embodiment is directed to a method of producing a photoelectric conversion element.

The method of producing a photoelectric conversion element of the present invention is a method of producing a photoelectric conversion element, the method comprising the steps of:
forming a first electrode;
forming a second electrode;
forming a photoelectric conversion layer containing a crystal having a perovskite structure between the first electrode and the second electrode; and
forming a charge-transporting layer between the photoelectric conversion layer and the first electrode,
wherein the charge-transporting layer contains a charge-transporting particle and an insulating resin, and is formed on a surface of the photoelectric conversion layer.

Each substance and each material are the same as those in the first embodiment, and hence description thereof is omitted.

As a result of investigations, the inventors of the present invention have found that a photoelectric conversion element having excellent conversion efficiency is obtained by using the above-mentioned production method. The reason therefor is conceived to be as described below.

The photoelectric conversion layer containing the crystal having a perovskite structure has unevenness on a surface of the crystal. In addition, a gap may be present between crystal grains. Deterioration tends to proceed when oxygen or the like is present in the gap, and hence it is preferred to fill the unevenness or the gap with a charge-transporting layer or the like. However, when a low-resistance charge-transporting layer is formed on the surface of the photoelectric conversion layer containing the crystal having a perovskite structure, the surfaces up to the side surfaces of the unevenness and the gap are covered with a low-resistance material with respect to the flow of charge. As a result, a state in which an electron and a hole easily recombine is established, and the state may lead to a reduction in conversion efficiency of the photoelectric conversion element.

In addition, when a high-resistance charge-transporting layer is formed on the surface of the photoelectric conversion layer containing the crystal having a perovskite structure, the exchange of charge is hindered, and hence the conversion efficiency of the photoelectric conversion element may reduce.

Meanwhile, when a charge-transporting layer is formed of a charge-transporting particle and an insulating resin on a perovskite crystal, the charge-transporting particle and the insulating resin are present independently. Accordingly, the insulating resin preferentially penetrates into the side surfaces of the unevenness and gap of the crystal having a perovskite structure, and hence the recombination of an electron and a hole occurring there is suppressed.

At the same time, it is conceived that, when the charge-transporting particle is present on the perovskite crystal, charge transportation efficiency from the perovskite crystal to the first electrode is maintained without the insulating resin becoming an obstacle to charge transportation.

### <Step of forming First Electrode and Step of forming Second Electrode>

The method of producing a photoelectric conversion element of the present invention includes the steps of: forming the first electrode; and forming the second electrode. In the step of forming the first electrode and the step of forming the second electrode, an appropriate method may be selected in accordance with a material for the first electrode and a material for the second electrode, respectively. Examples of such method include, but are not limited to, a sputtering method, a vacuum vapor deposition method, a vapor phase growth method (CVD method), and a spray pyrolysis deposition method (SPD method). The materials for the first electrode and the second electrode are as described above. When one, or each of both, of the first electrode and the second electrode is a transparent electrode, the thickness of the transparent electrode is preferably 0.03 to 3 µm.

When a solar cell is produced, cutting processing is generally performed for circuit formation between steps. Examples of the cutting processing include mechanical patterning and laser patterning.

### <Modularization Step>

The method of producing a photoelectric conversion element of the present invention may include a modularization step of sealing the element formed up to the electrode. A method for the sealing is, for example, sealing with a resin or sealing with a film. Examples of a material used for the sealing include a silazane, a silicone rubber, a resin having a siloxane skeleton, and glass.

In addition, hairline treatment may be performed on the surface of the sealed element from the viewpoint of the suppression of adhesion between elements occurring during winding in a roll-to-roll system.

### <Step of forming Photoelectric Conversion Layer>

The method of producing a photoelectric conversion element of the present invention comprises the step of forming the photoelectric conversion layer containing the crystal having a perovskite structure between the first electrode and the second electrode. The step of forming the photoelectric conversion layer comprises a step of applying a liquid containing the material for the photoelectric conversion layer described above. Examples of a method for the application include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or under an inert atmosphere (under a nitrogen or argon atmosphere) in order to remove a solvent or a dispersion medium from the applied liquid containing the material for the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a short-circuit current can be increased.

### <Step of forming Charge-transporting Layer>

The method of producing a photoelectric conversion element of the present invention comprises the step of forming the charge-transporting layer between the photoelectric conversion layer and the first electrode. In addition, the charge-transporting layer contains the charge-transporting particle and the insulating resin, and is formed on the surface of the photoelectric conversion layer.

The step of forming the charge-transporting layer is preferably a method of applying a resin solution in which the insulating resin is dissolved. Thus, the insulating resin preferentially penetrates into a gap between perovskite crystal grains with ease.

In addition, examples of the step of forming the charge-transporting layer include: a method including arranging the charge-transporting particle on the surface of the photoelectric conversion layer, and then applying the resin solution in which the insulating resin is dissolved; a method including applying the resin solution in which the insulating resin is dissolved on the surface of the photoelectric conversion layer, and then arranging the charge-transporting particle thereon; and a method including applying a solution, which is obtained by dispersing the charge-transporting particle in the resin solution in which the insulating resin is dissolved, onto the surface of the photoelectric conversion layer.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### (Example 1)

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1 (specific gravity: 1.6).

### <Production of Resin Solution 1>

1.0 Gram of polyvinyl butyral (product name: BM-2, manufactured by Sekisui Chemical Co., Ltd., specific gravity: 1.6) was dissolved in 19 g of 2-propanol by stirring for 24 hours to provide a resin solution 1.

### [Formation of Electron-transporting Layer]

A 25 mm×25 mm square glass substrate with ITO was washed, and a tin(II) oxide colloidal solution (15% water dispersion, manufactured by Alfa Aesar) diluted fivefold was applied thereonto by spin coating, followed by heating at 150°C for 30 minutes to form an electron-transporting layer as a thin film having a thickness of 16 nm.

### [Formation of Photoelectric Conversion Layer]

0.487 Gram of lead bromide, 1.034 g of formamidinium iodide, 2.903 g of lead iodide, and 0.139 g of methylammonium bromide were dissolved in 4.25 g of N,N-dimethylformamide and 1.216 g of dimethyl sulfoxide, and the solution was stirred for 1 hour (solution 1). Further, 0.100 g of cesium iodide was dissolved in 0.285 g of dimethyl sulfoxide, and the solution was stirred for 1 hour (solution 2). After that, the cesium iodide solution (solution 2) was added to the solution 1 to prepare a photoelectric conversion layer coating liquid. The coating liquid was applied onto the electron-transporting layer by spin coating in accordance with a poor solvent method to form a photoelectric conversion layer formed of Cs_{0.05}(FA_{0.83}MA_{0.17})_{0.95}Pb(I_{0.83}Br_{0.17})₃, the layer having a thickness of 600 nm and a surface roughness Ra of 13 nm.

### [Formation of Charge-transporting Layer]

0.1 Gram of the particle 1 and 0.01 g of a calixarene compound (Japanese Patent Laid-Open No. 2003-207913) were mixed with 10.6 g of 2-propanol, and 11 g of beads (zirconia beads, Torayceram (trademark) zirconia beads, 0.3 mm) were loaded into the mixture, followed by dispersion with a paint shaker (manufactured by Toyo Seiki Co., Ltd.) for 7 hours. After that, 0.2 g of the resin solution 1 was added thereto, and the mixture was dispersed with the paint shaker again for 6 hours to prepare a charge-transporting layer solution. The charge-transporting layer solution was applied onto the photoelectric conversion layer by spin coating to form a charge-transporting layer having a thickness of 160 nm.

### [Introduction of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium-bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a material solution for a second charge-transporting layer. The material solution was applied onto the above-mentioned charge-transporting layer by a spin coating method to form a second charge-transporting layer having a thickness of 100 nm.

### [Formation of First Electrode]

Ten gold electrodes each having a thickness of 80 nm and an area of 0.09 cm² were formed on the second charge-transporting layer by a vacuum vapor deposition method. Thus, a photoelectric conversion element was obtained.

### [Analysis of Compound Amount]

The electrode surface of the photoelectric conversion element was peeled off so that the surface of the charge-transporting layer was exposed. The surface of the charge-transporting layer was wiped with a cotton swab or the like with a solvent, dissolved in deuterated sulfuric acid, and subjected to ¹H-NMR measurement (apparatus: AVANCE III-500, manufactured by BRUKER). In addition, the peeled-off charge-transporting layer components were subjected to mass and structure analyses through GPC, MALDI-TOF-MS, IR, gas chromatography, or elemental analysis, such as XPS or EDX, to determine the presence of a compound.

In addition, the layer thickness was determined with a cross-sectional SEM (apparatus: SmartSEM, manufactured by Carl Zeiss Co., Ltd.) after the cutting of the photoelectric conversion element and the fixing of the sample to a tilted sample stage.

### [Measurement of Particle Diameter of Charge-transporting Particle]

The particle diameter of the charge-transporting particle in the charge-transporting layer is a volume-average particle diameter determined from a particle size distribution. In the present invention, the particle size distribution of the charge-transporting particles was derived by an image imaging method using a transmission electron microscope (TEM).

Specifically, first, N particles (N represents 1,000 or more) were extracted by using image processing software Photoshop (manufactured by Adobe Inc.) through use of a TEM image of the resultant cross section of the charge-transporting layer. Next, the area S of each particle was determined, the diameter of a circle with the same area as the area (=2×(S/π)^{1/2}) was defined as the particle diameter, and an average value of the central 80% of the N particles was adopted.

### [Measurement of Surface Roughness Ra of Photoelectric Conversion Layer]

The surface roughness Ra of the photoelectric conversion layer is the roughness of the surface on the first electrode side. A measurement method therefor is, for example, a method of subjecting the photoelectric conversion layer after the removal of the first electrode and the charge-transporting layer from the photoelectric conversion element to measurement with an AFM/SPM, or a method of calculating the surface roughness from a cross-sectional image of the photoelectric conversion element.

In this Example, the surface roughness Ra was calculated by image analysis of an image obtained as a height image in an AM-FM mode with an atomic force microscope AFM/SPM (MFP-3D Origin, manufactured by Oxford Instruments plc). As measurement conditions, a cantilever OMCL-AC-160TS (manufactured by Olympus Corporation) was used, and measurement was performed in the range of 90 µm×90 µm at a scanning frequency of 1 Hz. In addition, the number of X data was set to 256 and the number of Y data was set to 256.

### (Example 2)

1 Gram of the particle 1 obtained in the particle production step is mixed with 100 g of 2-propanol, 80 g of zirconia beads are loaded into the mixture, and the mixture is dispersed with a paint shaker (manufactured by Toyo Seiki Seisaku-sho, Ltd.) for 6 hours to produce a particle dispersion liquid. Then, the particle dispersion liquid is subjected to centrifugation (15,000 rpm, 3 minutes) with a tabletop high-spin centrifuge (D3024, manufactured by DLAB Scientific Co., Ltd.) so that the particle diameter in the dispersion liquid was reduced, followed by filtration and drying to provide a particle 2 having a reduced particle diameter. A photoelectric conversion element is obtained in the same manner as in Example 1 except that, in the formation of the charge-transporting layer, the particle 2 is used instead of the particle 1.

### (Example 3)

1 Gram of the particle 1 obtained in the particle production step is mixed with 100 g of 2-propanol, 80 g of zirconia beads are loaded into the mixture, and the mixture is dispersed with a paint shaker (manufactured by Toyo Seiki Seisaku-sho, Ltd.) for 10 hours to produce a particle dispersion liquid. Then, the particle dispersion liquid is subjected to centrifugation (15,000 rpm, 5 minutes) so that the particle diameter in the dispersion liquid was reduced, followed by filtration and drying to provide a particle 3 having a reduced particle diameter. A photoelectric conversion element is obtained in the same manner as in Example 1 except that, in the formation of the charge-transporting layer, the particle 3 is used instead of the particle 1.

### (Example 4)

A photoelectric conversion element is obtained in the same manner as in Example 1 except that, in the formation of the charge-transporting layer, the particle diameter in the dispersion liquid is increased by changing the dispersion time of the paint shaker dispersion from 6 hours to 4 hours.

### (Example 5)

A photoelectric conversion element is obtained in the same manner as in Example 1 except that, in the formation of the charge-transporting layer, the particle diameter in the dispersion liquid is increased by changing the dispersion time of the paint shaker dispersion from 6 hours to 2 hours.

### (Example 6)

A photoelectric conversion element is obtained in the same manner as in Example 1 except that the ratio of the volume of the charge-transporting particle to the volume of the insulating resin is set to 3.

### (Example 7)

A photoelectric conversion element is obtained in the same manner as in Example 1 except that the ratio of the volume of the charge-transporting particle to the volume of the insulating resin is set to 5.

### (Example 8)

A photoelectric conversion element is obtained in the same manner as in Example 1 except that the ratio of the volume of the charge-transporting particle to the volume of the insulating resin is set to 20.

### (Example 9)

A photoelectric conversion element is obtained in the same manner as in Example 1 except that the ratio of the volume of the charge-transporting particle to the volume of the insulating resin is set to 30.

### (Example 10)

A photoelectric conversion element is obtained in the same manner as in Example 1 except that the ratio of the volume of the charge-transporting particle to the volume of the insulating resin is set to 35.

### (Example 11)

A photoelectric conversion element is obtained in the same manner as in Example 1 except that the polyvinyl butyral (product name: BM-2, manufactured by Sekisui Chemical Co., Ltd.) is changed to polyvinyl butyral (product name: BX-1, manufactured by Sekisui Chemical Co., Ltd.).

### (Example 12)

A photoelectric conversion element is obtained in the same manner as in Example 1 except that the particle 1 is changed to a nickel phthalocyanine particle.

### (Example 13)

A photoelectric conversion element is obtained in the same manner as in Example 1 except that the particle 1 is changed to a copper phthalocyanine particle.

### (Example 14)

A photoelectric conversion element is obtained in the same manner as in Example 1 except that the second charge-transporting layer is not arranged.

### (Example 15)

A photoelectric conversion element is obtained in the same manner as in Example 1 except that the polyvinyl butyral is changed to polymethyl methacrylate (PMMA, manufactured by Sigma-Aldrich Co. LLC, glass transition temperature: 70°C).

### (Example 16)

A photoelectric conversion element is obtained in the same manner as in Example 1 except that the polyvinyl butyral is changed to polymethyl methacrylate (PMMA, manufactured by Sigma-Aldrich Co. LLC, glass transition temperature: 100°C).

### (Example 17)

A photoelectric conversion element is obtained in the same manner as in Example 1 except that the particle 1 is changed to a particle containing a compound represented by the following formula (Pc-3).

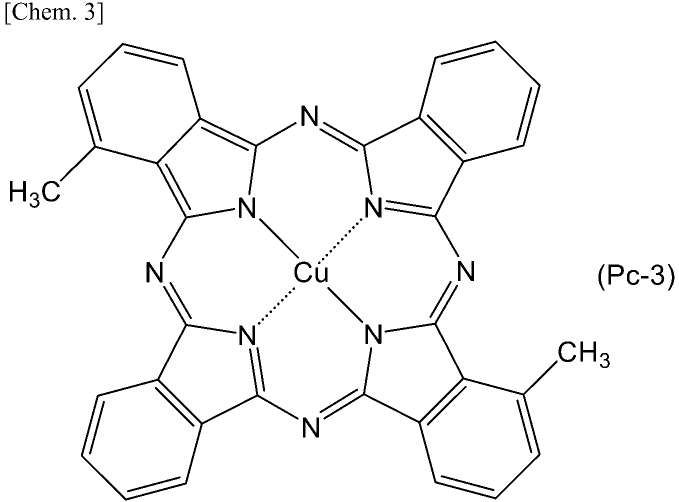

### (Example 18)

A photoelectric conversion element is obtained in the same manner as in Example 1 except that the particle 1 is changed to a quinacridone particle.

### (Example 19)

A photoelectric conversion element is obtained in the same manner as in Example 1 except that the particle 1 is changed to tetraphenylporphyrin (TPP).

### (Example 20)

A photoelectric conversion element is obtained in the same manner as in Example 1 except that a photoelectric conversion layer is formed without use of the poor solvent method. The surface roughness Ra of the photoelectric conversion layer was 122 nm.

### (Example 21)

A photoelectric conversion element is obtained in the same manner as in Example 1 except that a photoelectric conversion layer is formed without use of the poor solvent method by using 3.85 g of N,N-dimethylformamide and 1.15 g of dimethyl sulfoxide. The surface roughness Ra of the photoelectric conversion layer was 212 nm.

### (Comparative Example 1)

A photoelectric conversion element was obtained in the same manner as in Example 1 except that, in the formation of the charge-transporting layer, the particle 1 was not used.

### (Comparative Example 2)

A photoelectric conversion element is obtained in the same manner as in Example 1 except that the insulating resin is not used.

### (Comparative Example 3)

A photoelectric conversion element is obtained in the same manner as in Example 15 except that the particle 1 is changed to SPIRO-OMeTAD.

SPIRO-OMeTAD was compatible with the PMMA resin, and hence the SPIRO-OMeTAD was not able be observed as a particle.

### (Comparative Example 4)

A photoelectric conversion element is obtained in the same manner as in Example 1 except that P3HT is used as a conductive resin instead of the insulating resin.

### [Evaluation]

A power supply (236 model, manufactured by Keithley Instruments, LLC) is connected between the electrodes of the photoelectric conversion element produced in Example 1, and its photoelectric conversion efficiency is measured by: irradiating the element with constant light through use of a solar simulator (manufactured by Yamashita Denso Corporation) having an intensity of 114 mW/cm²; and measuring the generated current and voltage. In addition, each of the ten electrodes for each photoelectric conversion element is subjected to the measurement, and the average of the measured values is adopted as the representative value of the photoelectric conversion element. The results are shown in Table 2.

In Table 2, the photoelectric conversion efficiency in Example 1 is set to 100%, and a ratio thereto is shown as the conversion efficiency of each photoelectric conversion element.

### [Table 2]

**Table 2**

| | Charge-transporting material | | | Resin | | | Ratio of volume of charge-transporting material to volume of insulating resin in charge-transporting layer | Second charge-transporting layer | Photoelectric conversion efficiency [%] |
|---|---|---|---|---|---|---|---|---|---|
| | Name | Whether or not material is particle | Particle diameter [nm] | Insulating resin | Conductive resin | Glass transition temperature [°C] | | | |
| Example 1 | Hydroxygallium phthalocyanine | Particle | 2.1×10² | BM-2 | Absent | 71°C | 10 | Present | 100.0 |
| Example 2 | Hydroxygallium phthalocyanine | Particle | 1.3×10¹ | BM-2 | Absent | 71°C | 10 | Present | 95.8 |
| Example 3 | Hydroxygallium phthalocyanine | Particle | 8.0×10⁰ | BM-2 | Absent | 71°C | 10 | Present | 84.7 |
| Example 4 | Hydroxygallium phthalocyanine | Particle | 2.8×10² | BM-2 | Absent | 71°C | 10 | Present | 90.0 |
| Example 5 | Hydroxygallium phthalocyanine | Particle | 3.3×10² | BM-2 | Absent | 71°C | 10 | Present | 87.4 |
| Example 6 | Hydroxygallium phthalocyanine | Particle | 1.2×10² | BM-2 | Absent | 71°C | 3 | Present | 84.7 |
| Example 7 | Hydroxygallium phthalocyanine | Particle | 1.5×10² | BM-2 | Absent | 71°C | 5 | Present | 90.5 |
| Example 8 | Hydroxygallium phthalocyanine | Particle | 3.5×10² | BM-2 | Absent | 71°C | 20 | Present | 88.7 |
| Example 9 | Hydroxygallium phthalocyanine | Particle | 3.7×10² | BM-2 | Absent | 71°C | 30 | Present | 86.8 |
| Example 10 | Hydroxygallium phthalocyanine | Particle | 3.7×10² | BM-2 | Absent | 71°C | 35 | Present | 85.4 |
| Example 11 | Hydroxygallium phthalocyanine | Particle | 2.1×10² | BX-1 | Absent | 71°C | 10 | Present | 91.1 |
| Example 12 | Nickel phthalocyanine | Particle | 2.4×10² | BM-2 | Absent | 71°C | 10 | Present | 95.8 |
| Example 13 | Copper phthalocyanine | Particle | 1.6×10² | BM-2 | Absent | 71°C | 10 | Present | 95.3 |
| Example 14 | Hydroxygallium phthalocyanine | Particle | 2.2×10² | BM-2 | Absent | 71°C | 10 | Absent | 94.2 |
| Example 15 | Hydroxygallium phthalocyanine | Particle | 2.1×10² | PMMA | Absent | 70°C | 10 | Present | 92.1 |
| Example 16 | Hydroxygallium phthalocyanine | Particle | 2.1×10² | PMMA | Absent | 100°C | 10 | Present | 88.9 |
| Example 17 | (Pc-3) | Particle | 3.5×10¹ | BM-2 | Absent | 71°C | 10 | Present | 85.3 |
| Example 18 | Quinacridone | Particle | 1.3×10² | BM-2 | Absent | 71°C | 10 | Present | 80.5 |
| Example 19 | TPP | Particle | 1.7×10² | BM-2 | Absent | 71°C | 10 | Present | 83.2 |
| Example 20 | Hydroxygallium phthalocyanine | Particle | 2.1×10² | BM-2 | Absent | 71°C | 10 | Present | 95.5 |
| Example 21 | Hydroxygallium phthalocyanine | Particle | 2.1×10² | BM-2 | Absent | 71°C | 10 | Present | 88.4 |
| Comparative Example 1 | Absent | - | - | BM-2 | Absent | 71°C | 0 | Present | 23.7 |
| Comparative Example 2 | Hydroxygallium phthalocyanine | Particle | 1.5×10² | Absent | Absent | - | - | Present | 72.6 |
| Comparative Example 3 | SPIRO-OMeTAD | Not particle | - | PMMA | Absent | 100°C | 10 | Present | 65.8 |
| Comparative Example 4 | Hydroxygallium phthalocyanine | Particle | 1.5×10² | Absent | P3HT | - | 10 | Present | 66.8 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, and Japanese Patent Application No. 2024-086000 filed on May 28, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
11 perovskite crystal
12 charge-transporting particle
13 insulating resin
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer arranged between the photoelectric conversion layer and the first electrode, and
wherein the charge-transporting layer is formed of a charge-transporting particle and an insulating resin on a surface of the photoelectric conversion layer.

2. The photoelectric conversion element according to claim 1, wherein a surface roughness Ra of the crystal having a perovskite structure of the photoelectric conversion layer is 10 to 200 nm.

3. The photoelectric conversion element according to claim 1 or 2, wherein the insulating resin is arranged between the crystals each having a perovskite structure of the photoelectric conversion layer.

4. The photoelectric conversion element according to any one of claims 1 to 3, wherein an average particle diameter of the charge-transporting particle in the charge-transporting layer is 1.0×10¹ to 3.0×10² nm.

5. The photoelectric conversion element according to any one of claims 1 to 4, wherein a volume of the charge-transporting particle in the charge-transporting layer is 5 to 30 times with respect to a volume of the insulating resin in the charge-transporting layer.

6. The photoelectric conversion element according to any one of claims 1 to 5, wherein the charge-transporting particle is a particle containing a cyclic conjugated compound in which a plurality of pyrrole rings are bonded by conjugated bonds.

7. The photoelectric conversion element according to any one of claims 1 to 6, wherein a glass transition temperature of the insulating resin is 95°C or less.

8. The photoelectric conversion element according to any one of claims 1 to 7, wherein the charge-transporting particle is a particle containing a phthalocyanine compound.

9. The photoelectric conversion element according to any one of claims 1 to 8, wherein the charge-transporting particle is a particle having a structure represented by the following formula (Pc-2): 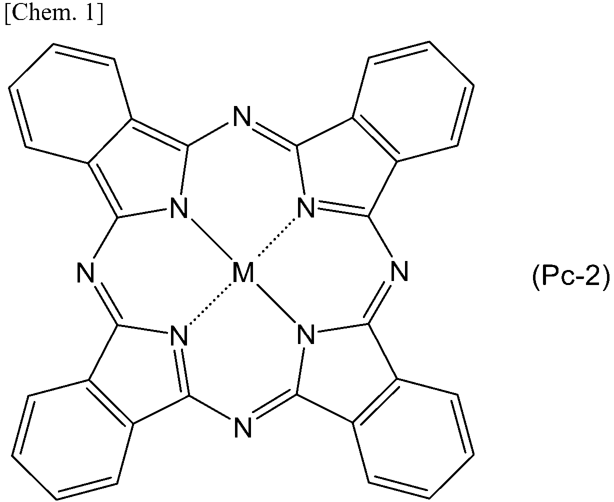 where M in the formula (Pc-2) represents H₂, a metal atom having a ligand, or a metal atom free of a ligand.

10. The photoelectric conversion element according to any one of claims 1 to 9, wherein the insulating resin is a polyvinyl acetal resin or a polyvinyl butyral resin.

11. The photoelectric conversion element according to any one of claims 1 to 10, wherein the charge-transporting layer contains an aromatic ring compound having a hydroxy group, the aromatic ring compound being different from a pigment or the insulating resin.

12. The photoelectric conversion element according to any one of claims 1 to 11, wherein the photoelectric conversion element comprises a second charge-transporting layer between the first electrode and the charge-transporting layer.

13. A photoelectric conversion apparatus comprising the photoelectric conversion element of any one of claims 1 to 12.

14. A method of producing a photoelectric conversion element, the method comprising the steps of:
forming a first electrode;
forming a second electrode;
forming a photoelectric conversion layer containing a crystal having a perovskite structure between the first electrode and the second electrode; and
forming a charge-transporting layer between the photoelectric conversion layer and the first electrode,
wherein the charge-transporting layer contains a charge-transporting particle and an insulating resin, and is formed on a surface of the photoelectric conversion layer.

15. The method of producing a photoelectric conversion element according to claim 14, wherein a surface roughness Ra of the crystal having a perovskite structure of the photoelectric conversion layer is 10 to 200 nm.

16. The method of producing a photoelectric conversion element according to claim 14 or 15, wherein an average particle diameter of the charge-transporting particle in the charge-transporting layer is 1.0×10¹ to 3.0×10² nm.

17. The method of producing a photoelectric conversion element according to any one of claims 14 to 16, wherein a volume of the charge-transporting particle in the charge-transporting layer is 5 to 30 times with respect to a volume of the insulating resin in the charge-transporting layer.

18. The method of producing a photoelectric conversion element according to any one of claims 14 to 17, wherein the charge-transporting particle is a particle containing a cyclic conjugated compound in which a plurality of pyrrole rings are bonded by conjugated bonds.

19. The method of producing a photoelectric conversion element according to any one of claims 14 to 18, wherein a glass transition temperature of the insulating resin is 95°C or less.

20. The method of producing a photoelectric conversion element according to any one of claims 14 to 19, wherein the charge-transporting particle is a particle containing a phthalocyanine compound.

21. The method of producing a photoelectric conversion element according to any one of claims 14 to 20, wherein the charge-transporting particle is a particle having a structure represented by the following formula (Pc-2): 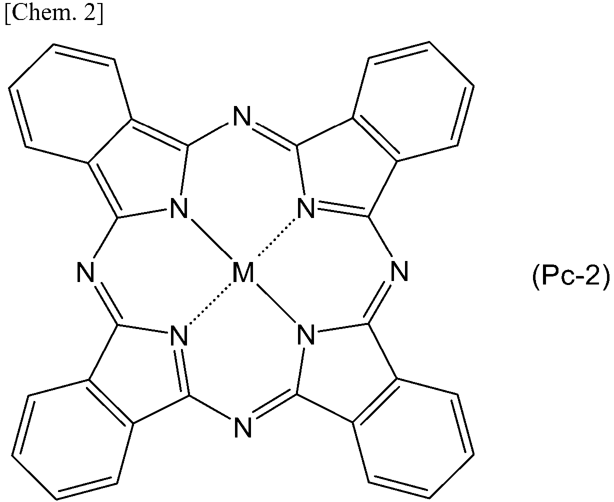 where M in the formula (Pc-2) represents H₂, a metal atom having a ligand, or a metal atom free of a ligand.

22. The method of producing a photoelectric conversion element according to any one of claims 14 to 21, wherein the insulating resin is a polyvinyl acetal resin or a polyvinyl butyral resin.

23. The method of producing a photoelectric conversion element according to any one of claims 14 to 22, wherein the charge-transporting layer contains an aromatic ring compound having a hydroxy group, the aromatic ring compound being different from a pigment or the insulating resin.

24. The method of producing a photoelectric conversion element according to any one of claims 14 to 23, wherein the photoelectric conversion element comprises a second charge-transporting layer between the first electrode and the charge-transporting layer.

## Amended claims

### Amended claims under Art. 19.1 PCT

1. (Amended) A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer arranged between the photoelectric conversion layer and the first electrode,
wherein the charge-transporting layer is formed of a charge-transporting particle and an insulating resin on a surface of the photoelectric conversion layer, and
wherein an average particle diameter of the charge-transporting particle in the charge-transporting layer is 1.0×10¹ to 3.0×10² nm.

2. The photoelectric conversion element according to claim 1, wherein a surface roughness Ra of the crystal having a perovskite structure of the photoelectric conversion layer is 10 to 200 nm.

3. The photoelectric conversion element according to claim 1 or 2, wherein the insulating resin is arranged between the crystals each having a perovskite structure of the photoelectric conversion layer.

4. (Deleted)

5. (Amended) The photoelectric conversion element according to any one of claims 1 to 3, wherein a volume of the charge-transporting particle in the charge-transporting layer is 5 to 30 times with respect to a volume of the insulating resin in the charge-transporting layer.

6. (Amended) The photoelectric conversion element according to any one of claims 1 to 3 and 5, wherein the charge-transporting particle is a particle containing a cyclic conjugated compound in which a plurality of pyrrole rings are bonded by conjugated bonds.

7. (Amended) The photoelectric conversion element according to any one of claims 1 to 3 and 5 to 6, wherein a glass transition temperature of the insulating resin is 95°C or less.

8. (Amended) The photoelectric conversion element according to any one of claims 1 to 3 and 5 to 7, wherein the charge-transporting particle is a particle containing a phthalocyanine compound.

9. (Amended) The photoelectric conversion element according to any one of claims 1 to 3 and 5 to 8, wherein the charge-transporting particle is a particle having a structure represented by the following formula (Pc-2): 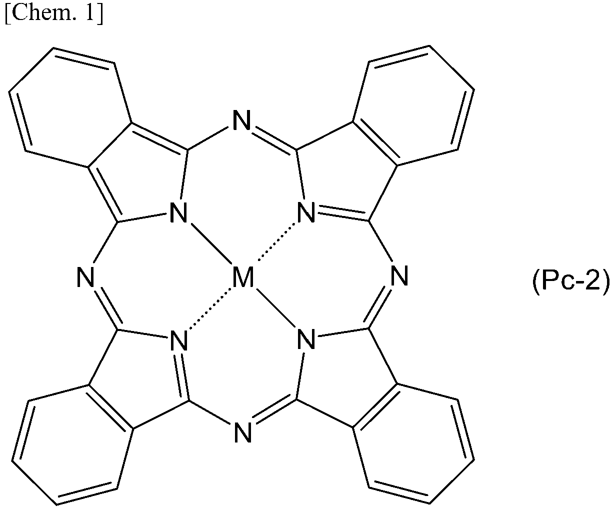 where M in the formula (Pc-2) represents H₂, a metal atom having a ligand, or a metal atom free of a ligand.

10. (Amended) The photoelectric conversion element according to any one of claims 1 to 3 and 5 to 9, wherein the insulating resin is a polyvinyl acetal resin or a polyvinyl butyral resin.

11. (Amended) The photoelectric conversion element according to any one of claims 1 to 3 and 5 to 10, wherein the charge-transporting layer contains an aromatic ring compound having a hydroxy group, the aromatic ring compound being different from a pigment or the insulating resin.

12. (Amended) The photoelectric conversion element according to any one of claims 1 to 3 and 5 to 11, wherein the photoelectric conversion element comprises a second charge-transporting layer between the first electrode and the charge-transporting layer.

13. (Amended) A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer arranged between the photoelectric conversion layer and the first electrode,
wherein the charge-transporting layer is formed of a charge-transporting particle and an insulating resin on a surface of the photoelectric conversion layer, and
wherein the charge-transporting particle is a particle containing a cyclic conjugated compound in which a plurality of pyrrole rings are bonded by conjugated bonds.

14. (Amended) A photoelectric conversion apparatus comprising the photoelectric conversion element of any one of claims 1 to 3 and 5 to 13.

15. (Amended) A method of producing a photoelectric conversion element, the method comprising the steps of:
forming a first electrode;
forming a second electrode;
forming a photoelectric conversion layer containing a crystal having a perovskite structure between the first electrode and the second electrode; and
forming a charge-transporting layer between the photoelectric conversion layer and the first electrode,
wherein the charge-transporting layer contains a charge-transporting particle and an insulating resin, and is formed on a surface of the photoelectric conversion layer, and
wherein an average particle diameter of the charge-transporting particle in the charge-transporting layer is 1.0×10¹ to 3.0×10² nm.

16. (Amended) The method of producing a photoelectric conversion element according to claim 15, wherein a surface roughness Ra of the crystal having a perovskite structure of the photoelectric conversion layer is 10 to 200 nm.

17. (Amended) The method of producing a photoelectric conversion element according to claim 15 or 16, wherein a volume of the charge-transporting particle in the charge-transporting layer is 5 to 30 times with respect to a volume of the insulating resin in the charge-transporting layer.

18. (Amended) The method of producing a photoelectric conversion element according to any one of claims 15 to 17, wherein the charge-transporting particle is a particle containing a cyclic conjugated compound in which a plurality of pyrrole rings are bonded by conjugated bonds.

19. (Amended) The method of producing a photoelectric conversion element according to any one of claims 15 to 18, wherein a glass transition temperature of the insulating resin is 95°C or less.

20. (Amended) The method of producing a photoelectric conversion element according to any one of claims 15 to 19, wherein the charge-transporting particle is a particle containing a phthalocyanine compound.

21. (Amended) The method of producing a photoelectric conversion element according to any one of claims 15 to 20, wherein the charge-transporting particle is a particle having a structure represented by the following formula (Pc-2): 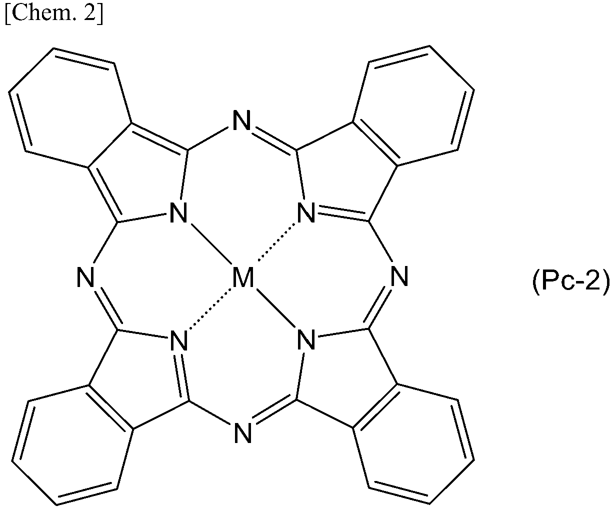 where M in the formula (Pc-2) represents H₂, a metal atom having a ligand, or a metal atom free of a ligand.

22. (Amended) The method of producing a photoelectric conversion element according to any one of claims 15 to 21, wherein the insulating resin is a polyvinyl acetal resin or a polyvinyl butyral resin.

23. (Amended) The method of producing a photoelectric conversion element according to any one of claims 15 to 22, wherein the charge-transporting layer contains an aromatic ring compound having a hydroxy group, the aromatic ring compound being different from a pigment or the insulating resin.

24. (Amended) The method of producing a photoelectric conversion element according to any one of claims 15 to 23, wherein the photoelectric conversion element comprises a second charge-transporting layer between the first electrode and the charge-transporting layer.
